# EUROPEAN PATENT APPLICATION

(11) **EP 4 424 303 A1**
(43) Date of publication of application: **04.09.2024**
(21) Application number: 23159148.8
(22) Date of filing: 28.02.2023
(51) Int. Cl.: A61K 9/16, A61K 9/48, A61K 31/47, A61K 31/00

(54) **LENVATINIB COMPOSITION WITH IMPROVED BIOAVAILABILITY**

(71) Applicant: STADA ARZNEIMITTEL AG, 61118 Bad Vilbel (DE); Zhejiang Qizheng Pharmaceutical Co.,Ltd., Taizhou City Zhejiang Province (CN)
(72) Inventor: CHEN, Hao, Zhejiang 317700 (CN); LIU, Jun, Zhejiang 317700 (CN); CHEN, Shuhua, Zhejiang 317700 (CN); ZHENG, Zhiguo, Zhejiang 317700 (CN); SCHÖNBORN, Jessica, 61118 Bad Vilbel (DE); WIEDERER, Andreas, 97320 Mainstockheim (DE); RENNER, Niklas, 61440 Oberursel (DE)
(74) Representative: Kernebeck, Thomas

(57) **Abstract**

The invention is directed to a pharmaceutical composition comprising a therapeutically effective dose of Lenvatinib salt and potassium carbonates, in particular potassium hydrogen carbonate, wherein the weight ratio of Lenvatinib salt to potassium carbonates is selected within a range from 1 : 0.3 to 1 : 3.0.

## Description

### Technical field

The present invention relates to a pharmaceutical composition comprising Lenvatinib salts, said pharmaceutical composition having improved bioavailability.

### Background of the present invention

Lenvatinib is an angiogenesis and C-Kit kinase inhibitor, and as such it is used as a therapeutic agent against various tumours such as thyroid cancer, lung cancer, melanoma and pancreatic cancer.

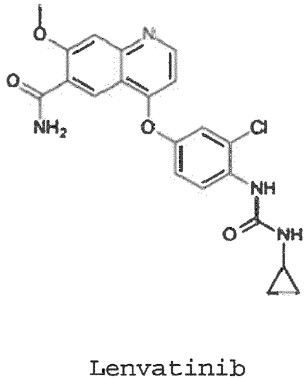

Lenvatinib free base has a low solubility in water and, therefore, is converted to different salts in order to improve its solubility. The marketed form of Lenvatinib is its mesylate salt. However, Lenvatinib mesylate degrades under humidifying and warm storage conditions when formulated into a pharmaceutical composition. Furthermore, it is known that Lenvatinib mesylate gelifies when in contact with dissolution media, this may cause a delay in its release.

To reduce degradation of the active substance, alkaline excipients are included in the pharmaceutical composition. Suitable alkaline excipients are for example sodium carbonates and alkaline earth metal carbonates such as magnesium carbonate and calcium carbonate.

In EP 2 468 281 A1 is described a pharmaceutical composition comprising:
(1) a compound represented by the formula (I) or pharmaceutically acceptable salt thereof or solvate thereof; 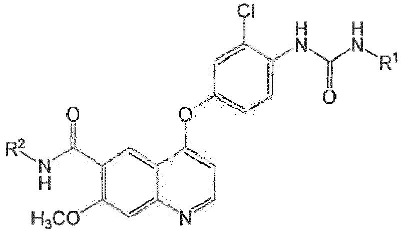 wherein R¹ is a hydrogen atom, a C₁₋₆ alkyl group or a C₃₋₈ cycloalkyl group; and R² represents a hydrogen atom or a methoxy group; and
(2) a basic substance.

Preferably, the basic substance is a carbonate, in particular an alkaline earth metal carbonate. Further preferred, the alkaline earth metal carbonate is a magnesium carbonate or a calcium carbonate.

In EP 3 632 436 is described a pharmaceutical composition comprising a therapeutically effective dose of Lenvatinib salt and sodium carbonates, preferably sodium bicarbonate, wherein the weight ratio of Lenvatinib salt to sodium carbonates ranges from 1: 1.5 to 1:10. Possible Lenvatinib salts are chloride, bromide, tosylate, sulphate, esylate, phosphate, tartrate, citrate, camphorsulphonate, isethionate, napadisylate, acetate, L-proline salt, maleate, salicylate, succinate, L-pyroglutamate, and besylate.

In WO 2018/185175 is described a pharmaceutical composition comprising a therapeutically effective dose of Lenvatinib mesylate and sodium carbonates, wherein the weight ratio of Lenvatinib mesylate to sodium carbonates ranges from 1 : 1.5 to 1 : 5.

In WO 2020/070147 is described a pharmaceutical composition comprising a therapeutically effective dose of Lenvatinib esylate or tosylate and sodium carbonates, wherein the weight ratio of Lenvatinib esylate or tosylate to sodium carbonates ranges from 1 : 1.5 to 1 : 10.

A further possibility to stabilize Lenvatinib is in the addition of a combination of potassium carbonate or potassium hydrogencarbonate and calcium hydrogen phosphate, calcium phosphate or calcium sulfate.

In CN 106 177 965 is described a pharmaceutical composition comprising as an active pharmaceutical ingredient 4-[3-chloro-4-(cyclopropylaminocarbonyl)aminophenoxy]-7-methoxy-6-quinolinecarboxamide or its pharmaceutically acceptable salt and
(A) at least one compound selected from the group consisting of potassium carbonate and potassium hydrogencarbonate;
(B) at least one compound selected from the group consisting of calcium hydrogen phosphate, calcium phosphate or calcium sulfate.

Preferably, the component (A) is potassium hydrogencarbonate.

The weight ratio of the components (A) and (B) is not particularly limited, and their weight ratio is selected between 1 : 0.1 and 1 : 10, preferably 1 : 0.5 to 1 : 2, most preferably 5 : 3.

The content of the components (A) and (B) is not limited, and as long as it contains a small amount, it can have an effect of improving dissolution and increasing stability. For the convenience of the formulation, the content of component (A) may range from 0.5% to 90%, preferably from 1% to 50%, more preferably from 1 to 35%, most preferably 5-20%, based on the total weight of the composition. The component (B) is present in an amount of from 0.5% to 90% by weight based on the total weight of the composition; preferably from 1% to 50%; more preferably from 1% to 25%; most preferably from 10% to 15%.

In the examples the pharmaceutical composition according to CN 106 177 965 is compared with a composition having a similar composition but only comprising potassium carbonate or potassium hydrogencarbonate and being void of calcium phosphate or calcium sulfate. The pharmaceutical compositions of comparative examples 3 and 4 contained the following ingredients (unit: mass%):

| Ingredient | Comparative Example 3 | Comparative Example 4 |
|---|---|---|
| Lenvatinib mesylate | 12.3 | 12.3 |
| Potassium carbonate | 20.0 | 0 |
| Potassium hydrogencarbonate | 0 | 20.0 |
| Weight ratio API/potassium carbonate | 0.615 | 0.615 |
| D-mannitol | 36.7 | 36.7 |
| Hydroxypropyl cellulose | 3.0 | 3.0 |
| Low-substituted hydroxypropyl cellulose | 25.0 | 25.0 |
| Talc powder | 3.0 | 3.0 |
| Total | 100 | 100 |

The dissolution rate of the capsules of Comparative Examples 3 to 4 was measured according to the second method (paddle method) of the dissolution test of the second edition of the Chinese Pharmacopoeia 2010 edition. 900 ml of a 0.1 mol/L hydrochloric acid solution was used as a dissolution medium, and a dissolution test was performed at 37 ± 0.5 ° C at a paddle speed of 50 rpm. As a result, in the capsules of Comparative Examples 3 to 4 containing potassium carbonate or potassium hydrogencarbonate in the formulation, the dissolution was quickly completed.

For evaluation of stability the capsules according to CN 106 177 965 and the capsules of Comparative Examples 3 and 4 were placed in an environment of a temperature of 60 ° C and a relative humidity of 75% and left in an open condition for 7 days. The samples were then analysed by HPLC to determine the formation of degradants.

In the samples according to CN 106 177 965 as well as in the capsules of Comparative Example 3 (containing potassium carbonate) and Comparative Example 4 (containing potassium bicarbonate) formation of the degradation product was not increased.

However, when analysing the dissolution of the samples after 7 days of storage the dissolution results showed that in the samples according to CN 106 177 965 the dissolution rate of Lenvatinib mesylate was good after 7 days standing, compared with the initial period. There was no significant drop and the dissolution was complete. However, in Comparative Example 3 (containing potassium carbonate) and Comparative Example 4 (containing potassium hydrogen carbonate), the dissolution rate of Lenvatinib mesylate decreased significantly after 7 days from the initial stage, and the dissolution was incomplete after 7 days of standing.

### Object of the invention

It is an object of the invention to provide a stable pharmaceutical composition comprising a Lenvatinib salt that has improved bioavailability and has high stability also after long-term storage.

### Solution to problem

The present inventors have intensively studied in order to solve the object described above and surprisingly have discovered the configuration below could solve the problems and have completed the present invention.

Specifically, the present invention provides a pharmaceutical composition comprising a therapeutically effective dose of Lenvatinib salt and potassium carbonates, wherein the weight ratio of Lenvatinib salt to potassium carbonates is selected within a range from 1 : 0.3 to 1 : 2.0.

The present inventors have found that by addition of a low amount of potassium carbonates gelification of the pharmaceutical composition can be supressed such that a quick disintegration of the composition at a pH as present in the gastrointestinal tract, in particular in the stomach, can be achieved. Further, the pharmaceutical is stable under long-term storage and quick dissolution is maintained.

### Advantageous Effects of Invention

The pharmaceutical composition of the present invention is excellent in dissolution of the Lenvatinib salt, which is the active ingredient. The pharmaceutical composition is also excellent in long-term stability. Quick disintegration of the pharmaceutical composition is prevented even after long term storage.

### Brief description of drawings

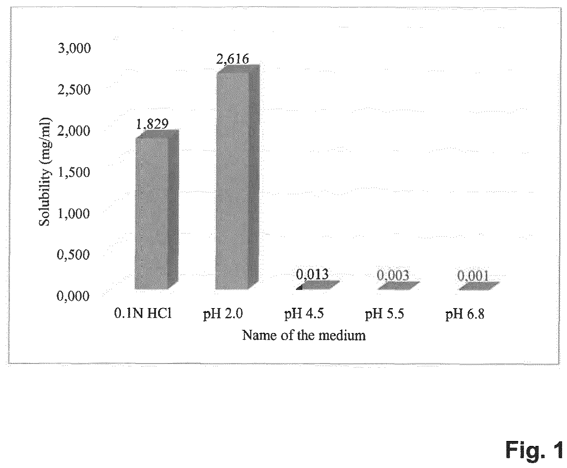

- Fig. 1:: is a diagram displaying the saturation solubility of Lenvatinib mesylate at 37±1°C.

### Detailed Description of the present Invention

According to the invention a pharmaceutical composition is provided comprising a therapeutically effective dose of Lenvatinib salt and potassium carbonates, wherein the weight ratio of Lenvatinib salt to potassium carbonates is selected within a range from 1 : 0.3 to 1 : 2.0.

According to a preferred embodiment, the weight ratio of Lenvatinib salt to potassium carbonates is selected within a range from 1 : 0.4 to 1 : 1.4, and according to a still further preferred embodiment, the weight ratio of Lenvatinib salt to potassium carbonates is selected within a range from 1 : 0.4 to 1 : 1.1, and according to a more preferred embodiment, the weight ratio of Lenvatinib salt to potassium carbonates is selected within a range from 1 : 0.4 to 1 : 0.9, and according to a most preferred embodiment, the weight ratio of Lenvatinib salt to potassium carbonates is selected within a range from 1 : 0.6 to 1 : 0.7.

Potassium carbonates within this invention encompass potassium carbonate (K₂CO₃), potassium hydrogen carbonate (KHCO₃), or mixtures of both. A preferred carbonate within the invention is potassium hydrogen carbonate.

In the acidic environment of the gastrointestinal tract, potassium carbonates produce carbon dioxide gas, which acts as a disintegrating agent and allows Lenvatinib salt to disperse in very small fine particles thereby avoiding gelation. The alkaline nature of the potassium carbonates prevents degradation and reduces impurity formation, including genotoxic impurities that may be formed upon hydrolysation of Lenvatinib salt.

Further, when the potassium carbonates are used in the ratio of the invention, the stability and the disintegration properties of these carbonates are such that Lenvatinib salt basically can be formulated without the addition of an extra disintegrant.

However, addition of an extra disintegrant is optional according to an embodiment.

Furthermore, potassium carbonates, and in particular KHCO₃, have good solubility in water compared to other earth metal carbonates thereby improving the pharmaceutical processability, i.e. by allowing wet granulation with water.

According to an embodiment, the pharmaceutical composition according to the invention does not comprise earth alkaline metal salts and, in particular, does not comprise calcium hydrogen phosphate, calcium phosphate or calcium sulfate.

Lenvatinib salts to be used according to the invention are mesylate, esylate, tosylate, besylate, phosphate, camphorsulphonate, isethionate, napadisylate, sulphate, bromide or chloride salt.

According to a preferred embodiment, the Lenvatinib salt is Lenvatinib mesylate.

According to an embodiment, the Lenvatinib salts comprised in the pharmaceutical composition according to the invention are having a particle size distribution D₉₀ from 5 to 50 µm, preferably from 8 to 25 µm, most preferred from 10 to 15 µm.

The D₉₀ value of the particle size distribution is defined as the particle diameter at which 90% by volume of the particles have a smaller diameter than the diameter which corresponds to the D₉₀ value measured by laser diffractometry. Specifically, a Malvern Instruments Mastersizer was used to determine the particle size distribution.

According to an embodiment, the potassium carbonates are present in a range from 6 to 12% by weight, based on the total weight of the composition, according to a further embodiment, the potassium carbonates are present in a range from 6.5 to 11.5% by weight, and, according to a still further embodiment, the potassium carbonates are present in a range from 7 to 11% by weight.

Besides potassium carbonates, in particular potassium hydrogen carbonate, one or more pharmaceutically acceptable excipients can be used additionally in accordance with the present invention.

The one or more pharmaceutically acceptable excipients to be used additionally to potassium carbonates, in particular potassium hydrogen carbonate, in accordance with the present invention can be chosen from, for example, diluents, binders, disintegrants, lubricants, and glidants.

Diluents are fillers which are used to increase the bulk volume of a tablet or capsule. By combining a diluent with the Lenvatinib salt, the final product is given adequate weight and size to assist in production and handling. Binders hold the excipients that are present in a tablet/granule together.

The pharmaceutical composition of the present invention preferably contains at least one diluent.

Diluents are preferably used in an amount of from 15% to 75%, preferably 30% to 70%, more preferably 35% to 65%, even more preferably 35% to 55% by weight based on the total weight of the composition. Suitable examples of diluents to be used in accordance with the present invention include starch, pregelatinized starch, microcrystalline cellulose (MCC), and mannitol.

In a preferred embodiment of the present invention, the diluents to be used are mannitol, microcrystalline cellulose or mixtures thereof.

The pharmaceutical composition of the present invention may also contain a binder. Binders ensure that tablets and granules can be formed having the desired or required mechanical strength. Binders which are suitable for use in accordance with the present invention include povidone, hydroxypropyl cellulose, hydroxypropyl methylcellulose, and sodium carboxyl methylcellulose. Binders are preferably used in an amount of from 1% to 6% by weight based on the total weight of the composition.

The pharmaceutical composition of the present invention may optionally contain an extra disintegrant. Disintegrants are added to a tablet or capsule composition to promote the breakup of the tablet/capsule into smaller fragments in an aqueous environment, thereby increasing the available surface area and promoting a more rapid release of the active pharmaceutical ingredient. Suitable examples of disintegrants to be used in accordance with the present invention include crospovidone, L-HPC (Low substituted hydroxypropyl cellulose), sodium starch glycolate, croscarmellose sodium, and mixtures of any of the foregoing. Extra disintegrants preferably are used in an amount of from 1% to 25% by weight based on the total weight of the composition; the amount will depend on the tablet size and the chosen disintegrant. A preferred extra disintegrant is low substituted hydroxypropyl cellulose, in a preferred amount of from 15% to 25% by weight based on the total weight of the composition.

The pharmaceutical composition of the invention may also contain a lubricant. Lubricants are generally used in order to reduce sliding friction. In particular, to decrease the friction at the interface between the blend to be encapsulated and dosator of the encapsulation machine. Suitable lubricants to be used in accordance with the present invention include magnesium stearate, stearic acid, glyceryl behenate, hydrogenated vegetable oil, talc and glycerine fumarate. A preferred lubricant is talc. The lubricant is preferably used in an amount of from 0.05% to 5% by weight based on the total weight of the composition, more preferred in an amount of 1.0% to 4.5% by weight, most preferred in an amount of 2.0% to 4.0% by weight, in particular 3% by weight.

The pharmaceutical composition of the invention may also contain a glidant. Glidants enhance product flow by reducing interparticulate friction. A suitable example is colloidal silicon dioxide.

Lubricants and glidants preferably are used in a total amount of from 0.05% to 5% by weight based on the total weight of the composition.

In a preferred embodiment, the pharmaceutical composition of the present invention, wherein the weight ratio of Lenvatinib salt to potassium carbonates, in particular potassium hydrogen carbonate, ranges from 1 : 0.3 to 1 : 3.0, more preferably from 1 : 0.8 to 1 : 1.9, most preferred ranges are from 1 : 0.9 to 1 : 1.8, contains the following ingredients, based on the total weight of the composition:
a) a therapeutically effective dose of Lenvatinib salt in an amount of from 4% to 30% by weight, preferably 4% to 25% by weight;
b) microcrystalline cellulose in an amount of from 10% to 65% by weight, 20% to 65% by weight, more preferably 25% to 55% by weight, even more preferably 27% to 45% by weight;
c) potassium carbonates, preferably potassium hydrogen carbonate, from 20% to 55% by weight, preferably from 20 to 50% by weight, more preferably 25 to 50% by weight;
d) optionally, Low substituted hydroxypropyl cellulose in an amount of from 15% to 25% by weight.
e) mannitol in an amount of from 7% to 18% by weight, preferably 5% to 10% by weight; and
f) from 1% to 5% by weight of a lubricant and a glidants, preferably talc.

In one embodiment of the present invention, the therapeutically effective dose of Lenvatinib is 4 mg, 10 mg, 18 mg and 24 mg.

The pharmaceutical composition of the present invention can be prepared by a method common in the art, for example, high shear wet granulation, dry granulation, one-step granulation, etc., to prepare granules of a pharmaceutical composition, and then filled into capsules to prepare a hard capsule.

In a preferred embodiment the pharmaceutical composition of the present invention is prepared by granulation process.

Granulation can be performed by a wet or dry process, wherein wet granulation using water or organic solvents or mixtures thereof as granulation liquid and dry granulation can be performed by processes known as slugging and/or roller compaction.

The pharmaceutically acceptable excipients to be used in accordance with the present invention, can be used only intragranularly, only extragranularly, or both.

According to a further embodiment, the pharmaceutical composition according to the invention is prepared by wet-granulation, which process comprises:
a. mixing Lenvatinib salt, potassium carbonates wherein the weight ratio of Lenvatinib salt to potassium carbonates ranges from 1 : 0.3 to 1 : 3.0;
b. add one or more pharmaceutically acceptable excipients to form a mixture;
c. wet-granulating the resulting mixture;
d. further mixing the obtained granulate with one or more further pharmaceutically acceptable excipients to form a further mixture;
e. Optionally encapsulating the granules

The granules of the present invention typically have a particle size distribution D₅₀ of from 200 µm - 350 µm, more preferably from 250 µm - 300 µm.

The present invention also relates to a pharmaceutical composition comprising granulates as described hereinabove in the form of a capsule or a tablet, preferably a capsule.

The pharmaceutical compositions described herein can be made using conventional methods and equipment well-known in the art.

The present invention is illustrated in more detail with reference to examples. However, it is understood that the invention is not limited to the examples.

### Examples

### Saturation solubility of Lenvatinib mesylate

The equilibrium solubility of Lenvatinib mesylate in different dissolution media at 37 °C ± 1 °C was measured by shake-flask technique. The dissolution media were prepared according to USP. The pH for each test solution was measured after the addition of the drug substance and at the end of the equilibrium solubility study.

The results are summarized in table 1 and are also displayed in fig. 1.

**Table 1: saturation solubility of Lenvatinib mesylate depending on pH**

| S.no | Name of the Medium | mg/ml | Initial pH | Final pH |
|---|---|---|---|---|
| 1 | 0.1N HCl | 1.829 | 1.1 | 1.23 |
| 2 | pH 2.0 | 2.616 | 2.0 | 1.88 |
| 3 | pH 4.5 | 0.013 | 4.52 | 4.38 |
| 4 | pH 5.5 | 0.003 | 5.49 | 5.25 |
| 5 | pH 6.8 | 0.001 | 6.82 | 6.68 |

### Manufacturing of Lenvatinib capsules-10 mg

The intragranular excipients (API, KHCO3, Mannitol, MCC PH 101, HPC, L-HPC) in the amounts as summarized in table 2 were loaded into a RMG (rapid mixer granulator) mixer, mixed for 10 minutes with impeller speed 500 rpm (rounds per minute). Subsequently, the chopper was turned off and water was sprayed as a binder onto the mixture. Mixing was continued for 1 min with an impeller speed of 500 rpm and chopper speed of 1000 rpm. Then the wet granules were dried by FBD (fluidized bed drying) with inlet temperature of 55 °C-65 °C until the granules LOD (Loss on drying) reach to below 2.0% ,

The dried granules were milled in a 1.0 mm mill.

The milled granules were introduced into a 1 L blender and the extragranular components in amounts mentioned as in table 2 (MCC (Avicel PH-102) and talc were added. The mixture was blended for 10 minutes at 20 rpm.

After blending the mixture was filled into Hydroxypropylmethylcellulose (HPMC) Size'4'shells.

**Table 2: Composition of Lenvatinib capsules (amounts in mg/unit)**

| Example | 1 | 2 |
|---|---|---|
| | | |

| **Intra-granular components** | | |
|---|---|---|
| Lenvatinib Mesylate* | 12.25 | 12.25 |
| KHCO₃ | - | 8.00 |
| Ratio Lenvatinib Mesylate to KHCO₃ | - | 0.65 |
| Mannitol | 8.75 | 8.75 |
| MCC (Avicel PH-101) | 43.00 | 45.00 |
| HPC | 3.00 | 3.00 |
| L-HPC | 25.00 | 15.00 |
| Purified water | 29.00 | 24.00 |
| Total Intragranular | 92.00 | 92.00 |

| **Extra-granular components** | | |
|---|---|---|
| MCC (Avicel PH-102) | 5.00 | 5.00 |
| Talc | 3.00 | 3.00 |
| Total Extragranular | 8.00 | 8.00 |
| Capsule fill weight | 100.00 | 100.00 |

12.25 mg Lenvatinib Mesylate correspond to 10 mg Lenvatinib.
- MCC:: micro-crystalline cellulose
- HPC:: Hydroxypropyl cellulose
- L-HPC:: low-substituted hydroxypropyl cellulose

### Dissolution of Capsules

A USP apparatus II (paddle) is used to test the dissolution profile with isa rotation speed of 50 rpm and900 ml 0.1 N HCl as a dissolution medium. The temperature is set to 37 °C ± 0.5 °C and the sampling times are 5 min, 10 min, 15 min, 20 min, 30 min, 45 min, 60 min and infinity (15 min with a rotation speed of 150 rpm)

The obtained data are summarized in table 3.

**Table 3: Dissolution profile of capsules obtained in examples 1 and 2, 0.1 N HCl; 900 ml; Paddle at 50 rpm speed; with sinker, 6 units have been tested**

| Time (min) | Example 1 | | Example 2 | |
|---|---|---|---|---|
| | Average % | RSD % | Average % | RSD % |
| 5 | 1 | 89.4 | 19 | 84.1 |
| 10 | 5 | 45.6 | 72 | 13.6 |
| 15 | 9 | 38.2 | 93 | 5.9 |
| 20 | 12 | 33.3 | 99 | 5.0 |
| 30 | 18 | 26.5 | 103 | 5.1 |
| 45 | 24 | 22.4 | 104 | 4.4 |
| 60 | 29 | 19.7 | 104 | 4.4 |
| Infinity | 56 | 9.7 | 104 | 4.4 |

| | | | | |
|---|---|---|---|---|
| RSD = relative standard deviation | | | | |

Formulation of example 1 (without KHCO₃) showed significantly less and no complete drug release compared to formulation of example 2 (containing KHCO₃) .

### Stress stability (60°C/75% RH) data:

Capsules obtained in examples 1 and 2 are packed in Polyamide-Alu-PVC pack and loaded in a climate chamber adjusted to 60°C/75% RH. After time periods indicated in table 4 a Polyamide-Alu-PVC pack was removed from the climate chamber and a capsule was taken from the pack. The capsule was opened and the ingredients were analysed by HPLC method(shown below). The total amount of impurities was determined by summing up the peak areas of the HPLC-chromatogram. Peak areas were calculated as percentage of the total area of peaks in the HPLC chromatogram.

The HPLC methods of related substances are as follows:

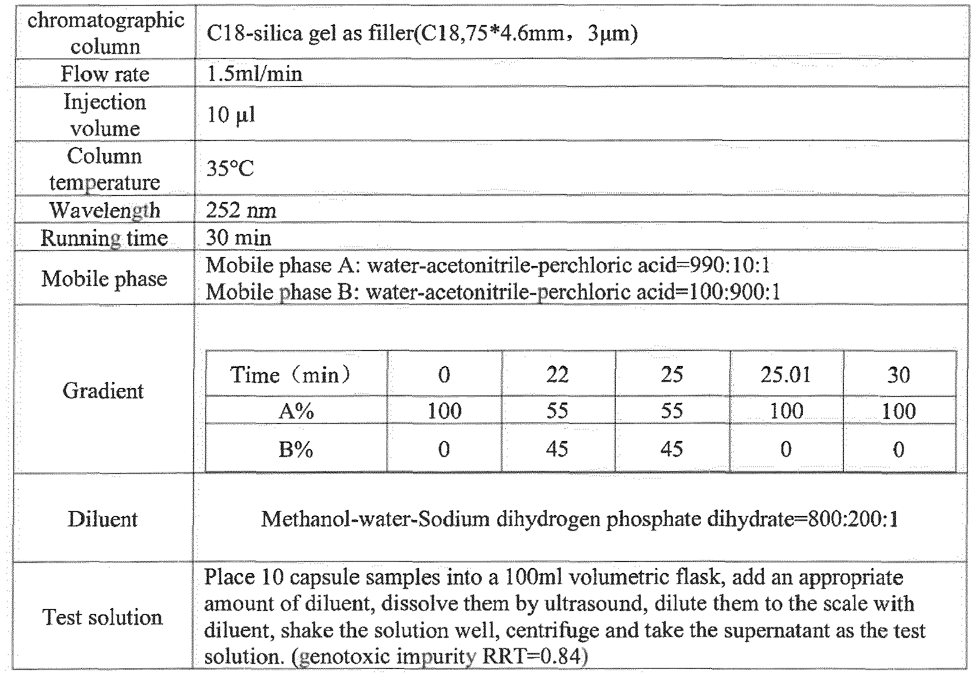

The data obtained are summarized in table 4. "Init" corresponds to the data obtained before introduction of the packs into the climate chamber. "Total imp" corresponds to the total impurities, which is the sum of all individual impurities.*)*

**Table 4: Stress test, amount of impurities (%) depending on time**

| | Example 1 | | | | Example 2 | | | |
|---|---|---|---|---|---|---|---|---|
| RRT | Init | 5day | 10day | | Init | 5day | 10day | |
| 0.31 | - | 0.005 | 0.008 | | - | - | - | |
| 0.43 | 0.015 | 0.370 | 0.645 | | 0.005 | 0.007 | 0.007 | |
| 0.44 | - | - | - | | 0.004 | 0.005 | 0.005 | |
| 0.45 | 0.014 | 0.016 | 0.017 | | - | - | - | |
| 0.58 | 0.018 | 0.019 | 0.019 | | 0.018 | 0.018 | 0.017 | |
| 0.66 | - | - | 0.004 | | - | - | - | |
| 0.70 | 0.057 | 0.069 | 0.077 | | 0.054 | 0.058 | 0.055 | |
| 0.80 | 0.029 | 0.029 | 0.029 | | 0.029 | 0.028 | 0.031 | |
| 0.82 | 0.004 | 0.005 | 0.004 | | 0.003 | 0.004 | 0.003 | |
| 0.85* | 0.019 | 0.035 | 0.048 | | 0.018 | 0.041 | 0.063 | |
| 0.87 | 0.007 | 0.053 | 0.088 | | - | - | - | |
| 0.90 | 0.022 | 0.022 | 0.023 | | 0.021 | 0.021 | 0.021 | |
| 0.93 | 0.021 | 0.020 | 0.021 | | 0.023 | 0.021 | 0.023 | |
| 1.03 | 0.002 | 0.002 | 0.002 | | 0.002 | 0.002 | 0.002 | |
| 1.04 | - | 0.006 | 0.009 | | 0.002 | 0.002 | 0.003 | |
| 1.08 | 0.004 | 0.022 | 0.033 | | 0.003 | 0.006 | 0.006 | |
| 1.15 | - | - | 0.005 | | - | 0.004 | 0.007 | |
| 1.17 | 0.008 | 0.007 | 0.007 | | 0.009 | 0.008 | 0.009 | |
| 1.24 | 0.006 | 0.005 | 0.005 | | 0.007 | 0.006 | 0.006 | |
| 1.32 | 0.017 | 0.017 | 0.016 | | 0.017 | 0.016 | 0.017 | |
| 1.43 | 0.009 | 0.009 | 0.008 | | - | - | - | |
| 1.44 | - | - | - | | 0.010 | 0.010 | 0.010 | |
| 1.51 | 0.016 | 0.015 | 0.016 | | 0.017 | 0.016 | 0.015 | |
| 1.55 | 0.010 | 0.008 | 0.010 | | 0.009 | 0.010 | 0.008 | |
| **Total imp.** | **0.278** | **0.732** | **1.092** | | **0.250** | **0.281** | **0.305** | |

| | | | | | | | | |
|---|---|---|---|---|---|---|---|---|
| RRT = relative retention time *RRT-0.5 is the genotoxic impurity | | | | | | | | |

The capsules of example 2 (formulation with KHCO₃) showed good stability. The amount of known/Unknown degradation products is within the ICH guideline Q3b(R2) limits.

The formulation of example 1 (without KHCO₃) showed more degradation. After storage in the climate chamber for 10 days a slight colour change to a light brown colour was observed.

### Impact of different quantities of KHCO3 per unit of Lenvatinib capsules-10 mg:

Capsules were prepared as described for examples 1 and 2 above. The amounts of excipients are summarized in table 5.

**Table 5:composition of capsules comprising Lenvatinib and varying amounts of KHCO₃**

| Example | 3 | 4 | 5 | 6 | 7 | 8 |
|---|---|---|---|---|---|---|
| | | | | | | |

| **Intra-granular components** | | | | | | |
|---|---|---|---|---|---|---|
| Lenvatinib Mesylate* | 12.25 | 12.25 | 12.25 | 12.25 | 12.25 | 12.25 |
| KHCO₃ | 33.00 | 25.00 | 12.50 | 8.00 | 8.00 | 8.00 |
| Ratio Lenvatinib Mesylate to KHCO₃ | 1 : 2.7 | 1 : 2 | 1 : 1 | 1 : 0.65 | 1 : 0.65 | 1 : 0.65 |
| Particle size* (KHCO₃) | D95=250 um | D95=25 0µm | D95=25 0µm | D95=25 0µm | D95=25 0µm | D95=250 µm |
| Mannitol | 8.75 | 8.75 | 8.75 | 8.75 | 8.75 | 8.75 |
| MCC (Avicel PH-101) | 10.00 | 18.00 | 30.50 | 35.00 | 45.00 | 45.00 |
| HPC | 3.00 | 3.00 | 3.00 | 3.00 | 3.00 | 3.00 |
| L-HPC | 25.00 | 25.00 | 25.00 | 25.00 | 15.00 | 15.00 |
| Purified water | 24.00 | 24.00 | 24.00 | 24.00 | 24.00 | 24.00 |
| Total Intragranu lar | 92 | 92 | 92 | 92 | 92 | 92 |

| **Extra-granular components** | | | | | | |
|---|---|---|---|---|---|---|
| MCC (Avicel PH-102) | 5.00 | 5.00 | 5.00 | 5.00 | 5.00 | 5.0 |
| Talc | 3.00 | 3.00 | 3.00 | 3.00 | 3.00 | 3.0 |
| Total Extragranu lar | 8.00 | 8.00 | 8.00 | 8.00 | 8.00 | 8.0 |
| Capsule fill weight | 100 | 100 | 100 | 100 | 100 | 100 |

| | | | | | | |
|---|---|---|---|---|---|---|
| *API psd is D90=27µm | | | | | | |

### Dissolution of capsules

Tests for dissolution of capsules obtained in examples 3 to 8 was performed as described above for examples 1 and 2. The data obtained are summarized in table 6:

**Table 6: Dissolution profile of capsules obtained in examples 3 to 8; 0.1N HCl; 900 ml; Paddle at 50rpm speed; with sinker;**

| Time (min) | Example 3 | Example 4 | Example 5 | Example 6 | Example 7 | Example 8 |
|---|---|---|---|---|---|---|
| 5 | 0 (0.0) | 1 (173.2) | 0 (0.0) | 8 (121.2) | 19 (84.1) | 0 (0.0) |
| 10 | 44 (23.9) | 45 (35.3) | 38 (79.2) | 69 (14.0) | 72 (13.6) | 12 (145.3) |
| 15 | 91 (1.3) | 90 (2.8) | 90 (3.2) | 89 (8.9) | 93 (5.9) | 79 (6.6) |
| 20 | 97 (3.3) | 97 (0.0) | 97 (2.6) | 97 (5.3) | 99 (5.0) | 93 (2.2) |
| 30 | 100 (2.6) | 100 (0.0) | 100 (2.3) | 101 (4.1) | 103 (5.1) | 100 (1.5) |
| 45 | 100 (2.5) | 100 (0.6) | 101 (2.9) | 102 (3.5) | 104 (4.4) | 102 (1.5) |
| 60 | 100 (2.5) | 100 (0.6) | 101 (2.6) | 102 (3.5) | 104 (4.4) | 103 (1.0) |
| Inf. | 100 (2.6) | 100 (0.6) | 101 (2.6) | 102 (3.5) | 104 (4.4) | 104 (1.0) |

As can be seen from the data presented in Table 6, the amount of KHCO₃ has limited impact on the dissolution of the capsules. **Influence of pH on dissolution of capsules**

Dissolution tests described above were repeated wherein, however, instead of 0.1 N HCl solution a solution was used wherein the pH of the solution was adjusted to pH 4.5 (Acetate buffer) or pH 6.8 (Phosphate buffer). In examples performed at a pH of 4.5 the paddle speed was adjusted to 100 rpm whereas in examples performed at a pH of 6.8 the paddle speed was adjusted to 50 rpm.

The concentration data obtained from samples taken after time periods as indicated are summarized in tables 7 and 8.

**Table 7: Dissolution profile of capsules obtained in examples 2 to 8 at pH = 4.5 buffer**

| Time (min) | Ex. 2 | Ex. 3 | Ex.4 | Ex.5 | Ex.6 | Ex.7* | Ex.8 |
|---|---|---|---|---|---|---|---|
| 5 | 12 (21.6) | 3 (145.3) | 1 (173.2) | 4 (104.1) | 22 (72.2) | 7 (8.7) | 1 (114.6) |
| 10 | 36 (19.3) | 22 (25.4) | 41 (19.9) | 46 (24.0) | 48 (21.5) | 31 (21.2) | 22 (39.3) |
| 15 | 49 (3.5) | 41 (6.2) | 62 (5.2) | 61 (11.0) | 61 (10.0) | 47 (16.9) | 35 (17.3) |
| 20 | 55 (2.1) | 60 (0.0) | 70 (2.9) | 67 (5.6) | 67 (6.5) | 58 (9.6) | 45 (8.5) |
| 30 | 60 (2.5) | 68 (0.0) | 75 (2.8) | 73 (2.8) | 73 (5.5) | 68 (5.3) | 57 (5.3) |
| 45 | 64 (2.7) | 72 (0.0) | 79 (2.2) | 78 (2.0) | 79 (4.8) | 75 (2.8) | 67 (3.8) |
| 60 | 67 (2.6) | 75 (0.8) | 82 (2.8) | 80 (2.6) | 82 (4.2) | 79 (1.9) | 72 (4.3) |
| Inf. | 76 (2.0) | 79 (0.7) | 85 (3.1) | 84 (1.8) | 86 (4.4) | 83 (2.1) | 78 (3.8) |

**Table 8: Dissolution profile of capsules obtained in examples 2, 7 and 8 at pH = 6.8 buffer**

| Time | Example 2 | Example 7* | Example 8 |
|---|---|---|---|
| 5 | 0 (173.2) | 1 (86.6) | 0 (0.0) |
| 10 | 1 (43.3) | 4 (35.3) | 1 (114.6) |
| 15 | 2 (0.0) | 6 (39.7) | 5 (72.1) |
| 20 | 3 (21.7) | 7 (37.8) | 6 (71.2) |
| 30 | 3 (17.3) | 8 (39.8) | 8 (65.7) |
| 45 | 4 (13.3) | 8 (3.8.6) | 9 (69.6) |
| 60 | 4 (13.3) | 9 (35.3) | 9 (69.7) |
| Infinity | 13 (9.1) | 19 (6.2) | 20 (11.4) |

| | | | |
|---|---|---|---|
| *: composition of capsules of example 7 is the same as in example 2; | | | |

### Dissolution of Lenvatinib capsules-10 mg on Stress stability (60°C/75% RH):

Dissolution tests were performed in 900 ml 0.1 N HCl as described above, however on capsules obtained according to examples 2 and 3 described above and stored in a climate chamber for 5 or 10 days as described above.

The data are summarized in table 9.

**Table 9: Dissolution profile of capsules obtained in examples 3 to 8 after storage in climate chamber (60°C/75% RH)**

| capsule | example 3 | | | example 2 | | |
|---|---|---|---|---|---|---|
| Time (min) | Initial | 5day | 10 day | Initial | 5day | 10day |
| 5 | 0 (0.0) | 2 (173.2) | 2 (173.2) | 1 (86.6) | 2 (107.9) | 1 (114.6) |
| 10 | 44 (23.9) | 57 (23.7) | 61 (11.0) | 46 (9.7) | 62 (14.3) | 50 (1.1) |
| 15 | 91 (1.3) | 88 (5.1) | 92 (3.8) | 76 (5.5) | 83 (9.2) | 75 (8.7) |
| 20 | 97 (3.3) | 96 (2.6) | 98 (2.7) | 87 (9.6) | 90 (8.2) | 82 (8.8) |
| 30 | 100 (2.6) | 99 (2.1) | 100 (3.2) | 94 (8.2) | 96 (6.5) | 89 (7.7) |
| 45 | 100 (2.5) | 100 (2.3) | 100 (3.2) | 100 (5.3) | 100 (4.1) | 93 (5.1) |
| 60 | 100 (2.5) | 100 (2.1) | 100 (3.2) | 102 (3.7) | 102 (2.0) | 96 (4.9) |
| Inf. | 100 (2.6) | 100 (1.7) | 100 (3.2) | 104 (3.6) | 104 (1.5) | 103 (1.7) |

Dissolution profile of stress stability samples are similar to initial dissolution (in 0.1 N HCl).

## Claims

1. A pharmaceutical composition comprising a therapeutically effective dose of Lenvatinib salt and potassium carbonates, wherein the weight ratio of Lenvatinib salt to potassium carbonates is selected within a range from 1 : 0.3 to 1 : 3.0.

2. The pharmaceutical composition according to claim 1, wherein the weight ratio of Lenvatinib salt to potassium carbonates is selected within a range from 1 : 0.4 to 1 : 1.4.

3. The pharmaceutical composition according to claim 1 or 2, wherein the Lenvatinib salt is Lenvatinib mesylate.

4. The pharmaceutical composition according to one of claims 1 to 3, wherein the potassium carbonates is potassium hydrogen carbonate.

5. The pharmaceutical composition according to one of claims 1 to 4, wherein potassium carbonates are present in a range from 6 to 12%, by weight based on the total weight of the composition.

6. A pharmaceutical composition according to any one of claims 1 to 5, wherein the composition further comprises:
a) at least one diluent in an amount of from 15% to 75% by weight, preferably 30% to 70% based on the total weight of the composition;
b) optionally, disintegrant in an amount of from 1% to 25% by weight based on the total weight of the composition; and
c) a lubricant in an amount of from 1 to 5% by weight based on the total weight of the composition.

7. A pharmaceutical composition according to claim 6 wherein the diluent is present in an amount of from 35% to 65% by weight based on the total weight of the composition.

8. A pharmaceutical composition according to claim 6 or 7 wherein the diluent is MCC, mannitol or a mixture of both.

9. A pharmaceutical composition according to any one of the previous claims comprising, based on the total weight of the composition,
a) A therapeutically effective dose of lenvatinib salt in an amount of from 4% to 25% by weight;
b) Microcrystalline cellulose in an amount of from 20% to 65% by weight;
c) potassium carbonates, preferably potassium hydrogen carbonate, in an amount of from 5% to 50 %, preferably 8% to 33% by weight;
d) Optionally, low substituted hydroxypropyl cellulose in an amount of from 15% to 25% by weight;
e) Mannitol in an amount of from 7% to 18%, preferably 5% to 10% by weight; and
f) Talc in an amount of from 1% to 5% by weight.

10. A pharmaceutical composition according to any one of claims 1 to 9 prepared by wet-granulation, which process comprises
a) mixing Lenvatinib salt, potassium carbonates, in particular potassium hydrogen carbonate, wherein the weight ratio of Lenvatinib salt to potassium carbonates ranges from 1 : 0.3 to 1 : 3.0.
b) add one or more pharmaceutically acceptable excipients to form a mixture;
c) wet-granulating the resulting mixture;
d) further mixing the obtained granulate with one or more further pharmaceutically acceptable excipients to form a further mixture;
e) optionally encapsulating the granules.

11. A pharmaceutical composition according to any one of the previous claims in the form of a capsule.
